# EUROPÄISCHE PATENTSCHRIFT

(11) **EP 1 729 703 B1**
(45) Veröffentlichungstag und Bekanntmachung des Hinweises auf die Patenterteilung: **15.08.2012**
(21) Anmeldenummer: 05715847.9
(22) Anmeldetag: 09.03.2005
(51) Int. Cl.: A61F 13/02

(54) **ELASTISCHES BANDAGESEGMENT**
ELASTIC BANDAGE SEGMENT
SEGMENT DE BANDAGE ELASTIQUE

(30) Priorität: 13.03.2004 DE 102004012442
(43) Veröffentlichungstag der Anmeldung: 13.12.2006
(73) Patentinhaber: Paul Hartmann AG, 89522 Heidenheim (DE)
(72) Erfinder: RITZDORF, Gerd, 56598 Hammerstein (DE); HILLE, Thomas, 56567 Neuwied (DE)
(74) Vertreter: Dreiss
(86) Internationale Anmeldenummer: PCT/EP2005/002451
(87) Internationale Veröffentlichungsnummer: WO 2005/087159

(56) Entgegenhaltungen:
- EP-A- 0 779 064
- WO-A-02/15816
- WO-A-99/16396
- DE-C1- 10 101 530
- GB-A- 350 842
- US-A- 4 424 808
- US-A1- 2003 040 691
- US-A1- 2003 139 697

## Beschreibung

Die Erfindung betrifft eine unidirektional elastische, einseitig haftklebende Bandage zur Stützung von Gelenken Außerdem betrifft die Erfindung ein Verfahren zur Herstellung derartiger Bandagesegmente sowie deren Verwendung.

Zur Vorbeugung und Minimierung von Verletzungen der Gelenke, wie z.B. Knie-, Sprung-, Hand-, Fingergelenken oder Teile des Bewegungsapparates werden stützende Verbände eingesetzt, die den wechselnden Anforderungen z.B. bei sportlichen Aktivitäten gerecht werden.

Bekannt sind klebende und nicht klebende, elastische Binden, wie z.B. das Handelsprodukt Porelastacryl, eine hautfarbene Pflasterbinde aus längselastischem Baumwollgewebe mit einem hypoallergenem Polyacrylathaftkleber beschichtet. Ein Nachteil dieser elastischen Binden besteht darin, dass sie, wenn nicht mit Haftkleber beschichtet, entweder durch die Bewegung nicht in der ursprünglichen Position verbleiben, verrutschen und somit ihre Funktion verlieren. Oder dass sie, wenn haftkleberbeschichtet, bei der Applikation zu Abschnürungen von Blutgefäßen führen können. Darüber hinaus ist das Anlegen dieser Binden für medizinisch ungeschultes Personal oftmals schwierig oder es erfordert eine spezielle Verbandstechnik.

EP 0 779 064 A1 offenbart einen chirurgischen Klebeverband, der sich unter leichter Dehnung der speziellen Kontur des Körperteils anpasst und der Bewegung der Anwendungsstelle folgen kann. Je länger die Zeit fortschreitet, umso weniger Gewicht wird benötigt, um die 50%-ige Dehnung in Position zu halten. Somit nimmt die Kraft des Materials zur Rückstellung in die ursprüngliche Lage ab. Der Klebeverband ist somit als plastisch zu bezeichnen (bleibende Verformung bei Einwirkung äußerer Kräfte).

US 2003/040691 A1 offenbart einen mehrlagigen elastischen Wundverband, geeignet zur Aufnahme von Wundsekret, mit mindestens drei Schichten. Der Wundverband besteht aus einer Kombination von elastischen und nichtelastischen Materialien. Der Wundverband wird um eine Wunde herum gewickelt und durch eine selbstklebende Ausrüstung, einem Klettverschluss, einem zusätzlichen Band oder ähnlichen Mitteln in Position gehalten. Dabei werden untere Lagen des Verbandes mit darüber liegenden Lagen verbunden.

WO 99/016396 beschreibt einen elastischen, klebenden Verband mit hoher Elastizität in Form eines Bumerangs, der auf Körperteile mit sehr ausgeprägten Krümmungen aufgebracht wird, ohne die Haut nach Aufbringen einer signifikanten Spannung auszusetzen. Damit soll die im wesentlichen Bildung von Falten nach Aufbringung verhindert werden. Durch die hohe Elastizität wird allerdings keine Stützung des Gelenks erreicht.

Aus der DE 101 01 530 C1 ist eine elastische Binde zum Fixieren von Auflagen und Abdeckungen bekannt, bestehend aus einem offenporigen, gitterartigen textilen Flächengebilde mit in Längsrichtung verlaufenden elastischen Fäden und in Querrichtung nicht oder wenig elastischen Fäden. Die Verbindung der Kett- und Schussfäden erfolgt an Kreuzungspunkten durch Klebemittel. Ein besonderes Merkmal dieser Binde ist das leichte Reißen in Längsrichtung. Die Binde weist eine Dehnbarkeit in Längsrichtung von größer/gleich 150% bis 350% auf. Zusätzlich ist eine einseitige Haftkleberbeschichtung aufgebracht, wobei das Material beim Anwickeln auf die Haut leicht anklebt. Ebenso haftet die Klebeseite leicht auf der nicht nichtklebenden Textilseite an.

US 2003/139697A1 offenbart einen dehnbaren Wundverband umfassend eine hydrokolloid Lage, die haftklebend ausgerüstet zur Aufnahme von Wundsekret geeignet ist. Durch parallele Anordnung der nicht-dehnbaren Verstärkungsteile wird eine Dehnung und Kontraktion in diese Richtungen verhindert, so dass die Ränder der Schnittwunde zusammengehalten werden. Der Wundverband ist durch drei Schichten aufgebaut: einer haftklebenden Schicht, einer Rückschicht und Verstärkungsteile.

WO 02/15816 A2 offenbart eine elastische, klebende Bandage mit einer absorbierenden Wundauflage zur Kontrolle von blutenden Wunden. Durch das Umwickeln des Körperteils wird ein hinreichende Kompressionsdruck aufgebaut, um die Bandage in Position zu halten. Die Bandage weist als zusätzliche Schicht eine integrierte Wundauflage auf.

US 4,424,808 A beschreibt eine in der Längsrichtung hochelastische und in der Schussrichtung leicht einreißbare, selbstklebende oder haftklebend ausgerüstete Breitbindgewebe mit Kreppstruktur. Die Binde klebt weder auf der Haut, Haar und Bekleidungsteilen, sondern sie haftet nach dem Umwickeln nur den einzelnen Touren auf sich selbst.

GB 350 842 A offenbart eine Wundverband mit einer Wundauflage, wobei der Verband in Querrichtung elastische Eigenschaften aufweist, wodurch die Wunde durch die Bandage zusammengehalten werden kann.

Aufgabe der Erfindung ist es deshalb, eine solche Stützbandage bereitzustellen, die die vorstehend genannten Nachteile vermeidet und einfach anzuwenden ist.

Erfindungsgemäß wird die Aufgabe durch ein Bandagesegment gemäß dem Anspruch 1 gelöst.

Ein solches Bandagesegment ist ein auf die Haut aufzubringendes Medizinprodukt, das das Aussehen traditioneller Pflaster aufweist. Zum Unterschied zu diesen wird es nicht auf offene Wunden geklebt. Dadurch, dass das Bandagesegment nicht wie herkömmliche Bandagen um das Gelenk gewickelt wird, kann es zu keiner Abschnürung kommen. Gleichzeitig wird durch das elastische Verhalten des Bandagesegments das Gelenk - auch bei Bewegungen - dauerhaft gestützt. Ein besonderer Vorteil der Bandagensegmente ist, dass diese auch von medizinisch nicht vorgebildeten Personen einfach zu applizieren ist und sich insbesondere bei längerem Tragen, auch beim Freizeitsport, kein Fremdkörpergefühl einstellt.

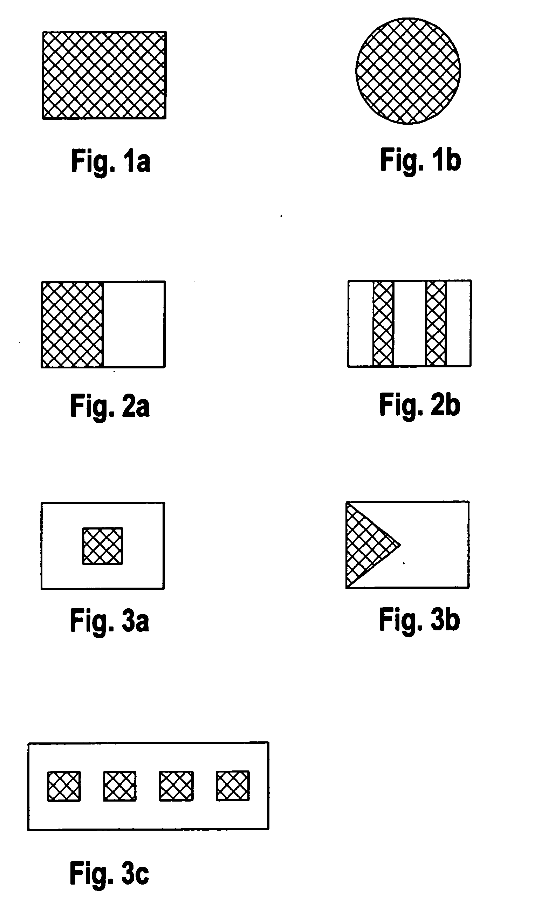

Das erfindungsgemäße Bandagesegment ist in den bevorzugten Ausführungsformen in Figuren 1-3 dargestellt, wobei die dargestellten Schraffierungen in den Zeichnungen die elastischen Bereiche wiedergeben.

Weitere bevorzugte Ausführungsformen sind Gegenstand der Unteransprüche. Vorzugsweise haben die Bandagensegmente eine rechteckige Gestalt und eine Größe mit einem Seitenverhältnis von Länge zu Breite von 1,2:1 bis 1,8:1, ähnlich wie z.B. Postkarten oder Scheckkarten, wie in Fig. 1a wiedergegeben. Aber auch runde Formen, wie in Fig. 1b dargestellt, bzw. der jeweiligen Anatomie angepasste Formen sind möglich.

Erfindungsgemäße Bandagesegmente, , sind Bandagesegmente, dargestellt in Fig. 2a und 2b, bei denen nur ein Teilbereich des Segments elastisch ausgestaltet ist. Z.B. sind nur ein mittlerer Bereich elastisch und die beiden Randbereiche nicht-elastisch ausgebildet. Weitere Ausführungsform erhält man, wenn zwei oder mehrere mittlere Bereiche elastisch und die beiden Randbereiche und die zwischen den elastischen Bereichen liegenden Bereichen nicht-elastisch ausgebildet sind. Damit erhält man ein Bandagesegment, das abwechselnd elastische und nicht-elastische Bereiche aufweist, wobei der

Abstand der elastischen Bereiche von der Anwendung abhängt. Durch diese Anordnung lässt sich sowohl über die Elastizität des Bandagesegments, als auch über die Größe der elastischen Bereiche der gewünschte Stützungseffekt erreichen.

Die elastischen Bereiche können auch so gestaltet sein, dass sie vollständig oder zumindest weitestgehend von nicht-elastischen Bereichen umgeben sind, wie in den
Figuren 3a, 3b, und 3c dargestellt. Die Form der elastischen Bereiche ist beliebig und hängt von dem jeweilig zu stützenden Bereich ab. Eine vorteilhafte Ausführungsform des Bandagesegments zeigt Fig. 3c mit mehreren in einem definierten Abstand nebeneinander bzw. hintereinander angeordneten elastischen Bereichen zur gleichzeitigen Stützung aller Gelenke z.B. zwischen den Mittelhandknochen und den Fingerknochen.

Die Elastizität wird bei der erfindungsgemäßen Bandage gemäß den üblicherweise bei Elastizitätsprüfungen verwendeten DIN Normen 60000 und 61632 (in der Version vom April 1985) bestimmt. Diese DIN Normen gelten zwar ursprünglich für Idealbinden; die für die Prüfung der Elastizität dabei verwendeten Horizontalkraftdehnungsanlage ist aber auch bei anderen Materialien analog einsetzbar.

Erfindungsgemäß ist bei der Bandage die Rückschicht nur in einer Richtung, d.h. in Längs- oder Querrichtung elastisch. Bezogen auf die Längsachse der Bandage ist die Gluerachse die im rechten Winkel dazu liegende Achse. Die andere Richtung der Rückschicht ist nicht elastisch. "Nicht-elastisch" bedeutet, dass bei Testung per Hand keine Elastizität feststellbar ist. Bei Messung gemäß DIN 61632 liegt dann die Elastizität unterhalb von 20 %. Erfindungsgemäß liegt also die Elastizität in einer - nämlich die elastische Richtung - oberhalb von 20 %.

Bevorzugt wird bei der erfindungsgemäßen Bandage ein elastisches Material für die Rückschicht verwendet, dessen Elastizität kleiner als 150 % ist. Bei einer stärker bevorzugten Ausführungsform liegt die Elastizität im Bereich von 20 - 80 %, besonders bevorzugt im Bereich zwischen 40 - 70 %. Am meisten bevorzugt und demzufolge für die Lösung der der Erfindung zugrunde liegenden Aufgabe am vorteilhaftesten ist ein Material für die Rückschicht, dessen Elastizität - immer gemessen DIN 61632 - im Bereich zwischen 44 und 56 % liegt.

Bevorzugte Materialien für die unidirektionale elastische Rückschicht sind mikrobiologisch nicht abbaubare Stoffe. Das Material sollte zu mehr als 90 %, bevorzugt zu mehr als 99 %, mikrobiologisch nicht abbaubar sein. Die Abbaubarkeit kann mit üblichen, dem Fachmann vertrauten Methoden, gemessen werden. Die geringe Abbaubarkeit ist besonders wichtig bei Medizinprodukten im dermalen Bereich, die länger auf der Haut getragen werden. Bedingt durch die Transpiration der Haut entsteht unmittelbar unter der durch Bandage abgedeckten Hautpartie ein Mikroklima, in dem Bakterien, Pilze, Sporen etc. prächtig gedeihen. Deshalb ist eine geringe mikrobiologische Abbaubarkeit, insbesondere bei längeren Tragezeiten, äußerst vorteilhaft. Darüber hinaus werden für die Rückschicht bevorzugt atmungsaktive und wasserdampfdurchlässige Materialien verwendet.

Bei dem Material der Rückschicht kann es sich um ein Gewebe, eine Folie oder eine Kombination aus beiden handeln, z.B. aus Viskose, Polyester, Polyamid, Baumwolle oder Elasthan. Umfasst die Rückschicht ein Polymer, so ist dieses vorteilhafterweise aus Polyethylen, Polypropylen oder Polyester insbesondere Polyakyllenterephthalaten ausgewählt.

Beispielhaft seien einige polymere Materialien für die Rückschicht genannt. Günstige polymere Materialien, die die vorstehenden Erfordernisse der geringen mikrobiologischen Abbaubarkeit erfüllen, sind Polyterephthalatsäurediester, die durch die Umsetzung der Ausgangsstoffe, ausgewählt aus Ethylenglykol, 1,4-Butandiol, 1,4-Dihydroxymethylcyclohexan, Terephthalsäure, Isophthalsäure, Adipinsäure, Acelainsäure, Sebacinsäure, Phthalsäure, Bisphenol-A-Diglycidylether, n-Decandicabonsäure-1,10, Polyethylenglykol und Polybuthylenglykol erhältlich sind.

Im Falle der Verwendung einer Folie liegt die Porosität im Bereich 10 - 50 %. Dabei bedeutet "Porosität" den Flächenanteil von Poren mit einer Fläche > 400 µm² an der jeweiligen Bezugsfläche. Diese relative Porenfläche kann durch Messung und Zählen der Poren einer beliebigen ungedehnten Bezugsfläche unter dem Mikroskop oder einem Fadenzähler ermittelt werden. Falls für die erfindungsgemäße Bandage ein Gewebe verwendet wird, weist die Rückschicht eine Kettfadenzahl im Bereich von 300 - 350, bevorzugt im Bereich von 310 - 330, und eine Schussfadenzahl im Bereich von 100 - 140, bevorzugt im Bereich 120 -130 jeweils gemessen je 10 cm ungedehntes Gewebe auf.

Die haftklebende Schicht besteht aus selbstklebenden Polymeren, ausgewählt aus der Gruppe der Polyacrylate, Silikone, Polyisobutylene und ähnlichen. Da das Bandagesegment durch den Kleber direkt auf der Haut fixiert wird, versteht es sich von selbst, dass die Klebkraft der Haftkleberschicht deutlich höher sein muss, als bei haftkleberbeschichteten, elastischen Binden, bei denen die Fixierung durch das Verkleben der Folien erfolgt. Besonders bevorzugt sind Haftkleberschichten, die auf einer Länge von 25 mm gemessen eine Klebkraft von 0,1 - 100 N besonders bevorzugt 1 - 10 N aufweisen, wobei der Haftkleber vollflächig, zumindest auf einem Teil der Unterseite des Trägers und/oder in Form von Mustern aufgebracht ist z.B. punkt- oder gitterförmig.

Die erfindungsgemäße Bandage wird mittels üblicher Verfahren hergestellt. Ein derartiges Verfahren umfasst im Allgemeinen die Schritte, dass ein silikonisiertes Papier mit einer haftkleberhaltigen Lösung beschichtet wird. Man entfernt eventuell vorhandenes Lösemittel durch Trocknen im Trockenkanal. Anschließend deckt man das Laminat, bestehend aus wieder ablösbarem Papier oder Folie und Haftkleberschicht, mit der undirektional elastischen Rückschicht ab.

An diesen Produktionsschritt kann sich ein Aufschneiden in Schmalrollen, aus denen die Segmente, mit dem Fachmann bekannten Verfahren, gestanzt oder geschnitten werden, anschließen. Es ist aber erfindungsgemäß auch möglich, die Herstellung der Segmente aus dem Laminat in Form der Breitrolle, dem Fachmann als so genannte Jumbo- oder Mutterrolle bekannt, durchzuführen. Anschließend werden die Bandagesegmente mit einem geeigneten Stanzwerkzeug formatgestanzt, vereinzelt und in Faltschachteln verpackt.

Die Erfindung wird nachfolgend durch ein Ausführungsbeispiel erläutert:

Beispiel: Zur Herstellung der erfindungsgemäßen undirektionalen elastischen Rückschicht wurde ein Polyestergewebe mit den folgenden Merkmalen, in Tabelle 1 wiedergegeben, mittels der dem Fachmann bekannten Techniken hergestellt.

**Tabelle 1**

| Prüfmerkmale | Einheit | Soll | Min | Max | Mittelwert |
|---|---|---|---|---|---|
| Warenbreite | mm | 1580 | 800 | 1600 | 1580 |
| Flächengewicht (ungedehnt) | g/m² | 100 | 95 | 103 | 100 |
| (DIN 53854 + DIN 53884) | | | | | |
| Dehnung (längs) | % | - | - | - | - |
| (quer) | % | 50 | 46 | 52 | 48 |
| (DIN 61632) | | | | | |
| Kettfadenzahl 10 cm ungedehnt | | 320 | 310 | 330 | 324 |
| Schussfadenzahl 10 cm ungedehnt | | 125 | 124 | 126 | 124 |

Außerdem wurden
581 Kg Durotak 387-2051 (52 %ige Lösung),
48 Kg Ethanol und
0,6 Kg Aluminiumacetylacetonat unter Rühren homogenisiert.
   Es wurde ca. 18 Stunden lang mit 56 Umdrehungen/Min. gerührt. Anschließend erfolgt eine Prüfung auf Homogenität. War die Masse homogen, wurde sie bei abgeschaltetem Rührer stehengelassen. Dadurch wird die Kleberlösung von Luftblasen befreit.

Nach erfolgter Homogenisierung wurde die Klebermasse auf ein silikonisiertes Papier aufgestrichen. Durch Trocknen bei üblicherweise 35 - 80°C wurden die organischen Lösungsmittel entfernt. Anschließend wurde das Laminat aus silikonisiertem Papier und Haftkleberschicht mit einem unidirektional elastischem Polyestergewebe gemäß Tabelle 1 abgedeckt. Aus dem so erhaltenen Laminat wurden die Bandagesegmente mit den Maßen 60 x 90 mm auf Format gestanzt.

## Patentansprüche

1. Unidirektional elastische, einseitig haftklebende Bandage zur Stützung von Gelenken, **dadurch gekennzeichnet, dass** die Bandage als Segment vorliegt, wobei das Bandagensegment nicht um ein Gelenk gewickelt wird und der Haftkleber vollflächig oder auf einem Teil der Unterseite des Trägers und/oder in Form von Mustern aufgebracht ist und der Haftkleber bestehend aus selbstklebenden Polymeren ausgewählt ist aus der Gruppe der Polyacrylate, Silikone, Polyisobutylene und wobei ein oder mehrere definierte Bereiche des Segments elastisch ausgestaltet sind.

2. Unidirektional elastische, klebende Bandage nach Anspruch 1, **dadurch gekennzeichnet, dass** das Bandagesegment queroder längselastisch ist.

3. Unidirektional elastische, klebende Bandage nach Anspruch 1 bis 2, **dadurch gekennzeichnet, dass** die Elastizität der Rückschicht im Bereich von 20%-150%, bevorzugt zwischen 20%-80%, bevorzugt zwischen 40%-70%, besonders bevorzugt zwischen 44%-56% liegt.

4. Unidirektional elastische, klebende Bandage nach Anspruch 1 bis 3, **dadurch gekennzeichnet, dass** der Haftkleber eine Klebkraft von 0,1-100, 0 N, bevorzugt 1,0-10,0 N aufweist.

5. Unidirektional elastische, klebende Bandage nach einem der vorangehenden Ansprüche, **dadurch gekennzeichnet, dass** das Material der Rückschicht zu mehr als 90%, bevorzugt zu mehr als 99% mikrobiologisch nicht abbaubar ist.

6. Unidirektional elastische, klebende Bandage nach einem der vorangehenden Ansprüche, **dadurch gekennzeichnet, dass** die Rückschicht atmungsaktiv und wasserdampfdurchlässig ist.

7. Unidirektional elastische, klebende Bandage nach einem der vorangehenden Ansprüche, **dadurch gekennzeichnet, dass** das Material der Rückschicht ein Gewebe, eine Folie oder eine Kombination aus beiden aus Viskose, Polyester, Polyamid, Baumwolle oder Elasthan ist.

8. Unidirektional elastische, klebende Bandage nach Anspruch 7, **dadurch gekennzeichnet, dass** das Material der Rückschicht ein polymeres Material ausgewählt aus der Gruppe Polyethylen, Polypropylen, Polyester, insbesondere Polyalkylenterephthalaten ist.

9. Unidirektional elastische, klebende Bandage nach Anspruch 7 und 8, **dadurch gekennzeichnet, dass** die Porosität der Folie im Bereich von 10-50% liegt.

10. Unidirektional elastische, klebende Bandage nach Anspruch 7 und 8, **dadurch gekennzeichnet, dass** die Rückschicht eine Kettfadenzahl im Bereich von 300-350, bevorzugt 310-330 und eine Schussfadenzahl im Bereich von 100-140, bevorzugt 120-130 aufweist.

11. Unidirektional elastische, klebende Bandage nach einem der vorangehenden Ansprüche, **dadurch gekennzeichnet, dass** die Gestalt des Bandagesegments rechteckig mit einem Seitenverhältnis von Länge zu Breite von 1,2:1 bis 1,8:1 aufweist, bevorzugt die Grösse von 10 cm mal 8 cm hat.

12. Verfahren zur Herstellung von unidirektional elastischen, klebenden Bandagesegmenten nach den vorangehenden Ansprüchen umfassend die Schritte:
- Beschichten von silikonisiertem Papier/Folie mit haftkleberhaltiger Lösung,
- Abdecken des Laminats aus wiederablösbarem Papier/Folie und der Haftkleberschicht mit der undirektional elastischen Rückschicht und
- Formstanzen der Bandagesegmente aus Schmal- oder Breitrollen.

13. Verwendung von unidirektional elastischen, klebenden Bandagesegmenten nach den vorangehenden Ansprüchen zur Stützung von Gelenken.

## Claims

1. Unidirectionally elastic bandage, adhesive on one side and used for supporting joints, **characterized in that** the bandage is embodied as a segment, wherein the bandage segment is not wound round a joint and wherein the adhesive is applied across the entire surface of or on a part of the underside of the carrier and/or in the form of patterns and wherein the adhesive composed of self-adhesive polymers is chosen from the group of polyacrylates, silicones, polyisobutylenes and wherein one or more defined areas of the segment are made elastic.

2. Unidirectionally elastic adhesive bandage according to claim 1, **characterized in that** the bandage segment is transversely elastic or longitudinally elastic.

3. Unidirectionally elastic adhesive bandage according to claims 1 to 2, **characterized in that** the elasticity of the backing layer lies in the range of between 20% and 150%, and preferably in the range of between 20% and 80%, preferably between 40% and 70%, particularly preferably between 44% and 56%.

4. Unidirectionally elastic adhesive bandage according to claims 1 to 3, **characterized in that** the adhesive has an adhesion force of 0.1 - 100.0 N, preferably 1.0 - 10.0 N.

5. Unidirectionally elastic adhesive bandage according to one of the preceding claims, **characterized in that** the material of the backing layer is microbiologically nondegradable to an extent of more than 90%, preferably to an extent of more than 99%.

6. Unidirectionally elastic adhesive bandage according to one of the preceding claims, **characterized in that** the backing layer is breathable and allows water vapor to pass through.

7. Unidirectionally elastic adhesive bandage according to one of the preceding claims, **characterized in that** the material of the backing layer is a woven fabric, a film or a combination of both made form viscose, polyester, polyamide, cotton or elastane.

8. Unidirectionally elastic adhesive bandage according to claim 7, **characterized in that** the material of the backing layer is a polymer material chosen from the group of polyethylene, polypropylene, polyester, in particular polyalkylene terephthalates.

9. Unidirectionally elastic adhesive bandage according to claims 7 and 8, **characterized in that** the porosity of the film is in the range of 10 to 50%.

10. Unidirectionally elastic adhesive bandage according to claims 7 and 8, **characterized in that** the backing layer has a warp number in the range of 300 - 350, preferably 310 - 330, and a weft number in the range of 100 - 140, preferably 120 - 130.

11. Unidirectionally elastic adhesive bandage according to one of the preceding claims, **characterized in that** the shape of the bandage segment is rectangular with a side ratio of length to width of 1.2:1 to 1.8:1, preferably having a size of 10 cm by 8 cm.

12. Method for producing unidirectionally elastic adhesive bandage segments according to one of the preceding claims comprising the steps:
- coating silicone-treated paper/film with adhesive-containing solution,
- covering the laminate of releasable paper/film and adhesive layer with the unidirectionally elastic backing layer, and
- punching the bandage segments out from narrow or wide rolls.

13. Use of unidirectionally elastic adhesive bandage segments according to one of the preceding claims for supporting joints.

## Revendications

1. Bandage élastique de façon unidirectionnelle et unilatéralement auto-adhésif, destiné à soutenir des articulations, **caractérisé en ce que** le bandage se présente sous forme d'un segment, le segment de bandage n'étant pas enroulé autour d'une articulation et la colle étant appliquée sur toute la surface ou sur une partie de la face inférieure du support et/ou sous forme de motifs, et la colle constituée de polymères auto-adhésifs étant choisie dans le groupe constitué par les polyacrylates, les silicones, les polyisobutylènes et une ou plusieurs zones définies du segment étant conçues de façon élastique.

2. Bandage adhésif élastique de façon unidirectionnelle selon la revendication 1, **caractérisé en ce que** le segment de bandage est transversalement ou longitudinalement élastique.

3. Bandage adhésif élastique de façon unidirectionnelle selon la revendication 1 ou 2, **caractérisé en ce que** l'élasticité de la couche arrière se situe dans la plage de 20 % à 150 %, est comprise de préférence entre 20 % et 80 %, de préférence entre 40 % et 70 %, et de préférence encore entre 44 % et 56 %.

4. Bandage adhésif élastique de façon unidirectionnelle selon les revendications 1 à 3, **caractérisé en ce que** la colle présente une force d'adhérence de 0,1 à 100,0 N, de préférence de 1,0 à 10,0 N.

5. Bandage adhésif élastique de façon unidirectionnelle selon l'une quelconque des revendications précédentes, **caractérisé en ce que** le matériau de la couche arrière n'est pas microbiologiquement dégradable à plus de 90 %, de préférence à plus de 99 %.

6. Bandage adhésif élastique de façon unidirectionnelle selon l'une quelconque des revendications précédentes, **caractérisé en ce que** la couche arrière est perméable à l'air et à la vapeur d'eau.

7. Bandage adhésif élastique de façon unidirectionnelle selon l'une quelconque des revendications précédentes, **caractérisé en ce que** le matériau de la couche arrière est un tissu, un film ou une combinaison des deux composée de viscose, de polyester, de polyamide, de coton ou d'élasthanne.

8. Bandage adhésif élastique de façon unidirectionnelle selon la revendication 7, **caractérisé en ce que** le matériau de la couche arrière est un matériau polymère choisi dans le groupe polyéthylène, polypropylène, polyester, en particulier polyalkylentéréphtalate.

9. Bandage adhésif élastique de façon unidirectionnelle selon les revendications 7 et 8, **caractérisé en ce que** la porosité du film se situe dans la plage de 10 à 50 %.

10. Bandage adhésif élastique de façon unidirectionnelle selon les revendications 7 et 8, **caractérisé en ce que** la couche arrière comprend un nombre de fils de chaîne dans la plage de 300 à 350, de préférence de 310 à 330 et un nombre de fils de trame dans la plage de 100 à 140, de préférence de 120 à 130.

11. Bandage adhésif élastique de façon unidirectionnelle selon l'une quelconque des revendications précédentes, **caractérisé en ce que** le segment de bandage présente une forme de rectangle doté d'un rapport longueur sur largeur de 1,2:1 à 1,8:1, de préférence une taille de 10 cm sur 8 cm.

12. Procédé de fabrication de segments de bandage adhésif élastique de façon unidirectionnelle selon les revendications précédentes, comprenant les étapes consistant à:
- revêtir le papier/film siliconé de solution contenant une colle,
- recouvrir le stratifié composé du papier/film amovible et de la couche de colle avec la couche arrière élastique de façon unidirectionnelle et
- découper les segments de bandage à partir des rouleaux étroits ou larges.

13. Utilisation de segments de bandage adhésif élastique de façon unidirectionnelle selon les revendications précédentes pour le soutien des articulations.
